# EUROPEAN PATENT APPLICATION

(11) **EP 4 400 108 A1**
(43) Date of publication of application: **17.07.2024**
(21) Application number: 22864756.6
(22) Date of filing: 05.09.2022
(51) Int. Cl.: A61K 31/6615, A61K 47/26, A61K 47/36, A61K 47/38, A61P 3/06, A61P 3/10, A61P 9/00, A61P 13/02, A61P 19/06, A61P 43/00

(54) **WATER-SOLUBLE PHYTIN**

(30) Priority: 06.09.2021 JP 2021144782
(71) Applicant: Otsuka Pharmaceutical Co., Ltd., Tokyo 101-8535 (JP)
(72) Inventor: IKENAGA, Takeshi, Osaka-shi, Osaka 540-0021 (JP); SATO, Ikutaro, Osaka-shi, Osaka 540-0021 (JP); KOUDA, Noriyuki, Osaka-shi, Osaka 540-0021 (JP)
(74) Representative: Hoffmann Eitle
(86) International application number: PCT/JP2022/033286
(87) International publication number: WO 2023/033174

(57) **Abstract**

The present application provides a phytin with improved solubility in water, wherein the phytin comprises calcium and / or magnesium with specific of molar ratios to phytic acid.

## Description

### Technical Field

The present application relates to phytin with improved solubility in water and a composition comprising the same.

### Background Art

Phytic acid has chelating and antioxidant effects, and is also known for its anticoagulant, prevention of hypercalciuria, and lipid-improving effects. Further, Patent Document 2 discloses that phytic acid has a purine nucleotide metabolism inhibitory effect and a phosphatase inhibitory effect, and has an effect of suppressing increases in serum uric acid levels in humans. Furthermore, Patent Document 2 describes the blood pressure-improving and blood glucose-improving effects of phytic acid.

Phytic acid is usually in a liquid state, but it is commercially available as a powdered preparation, such as phytic acid powder product (50%), which is made by adding excipients and the like to phytic acid to make it easier to process. However, since the phytic acid powder product is deliquescent and has poor stability, efforts have been made to stabilize it, such as by creating a stabilized powdered phytic acid composition in which phytic acid is present in close contact with sodium acetate powder (Patent Document 3) .

On the other hand, phytin, a mineral (such as calcium and magnesium) salt of phytic acid is commercially available in powder form and remains stable without deliquescing. Phytin is a general term for the mineral salts, and the kinds or molar ratios of mineral components such as calcium and magnesium in phytin are not fixed; in fact, the molar ratios of mineral components such as magnesium and calcium in naturally occurring phytin in rice, corn, wheat, oats, etc., are different (Non-Patent Document 1). Although various pharmacological/health-promoting effects are expected from phytin due to its constituent phytic acid, conventionally-known phytins do not dissolve in water (Non-Patent Document 2), making them unsuitable for use in formulations requiring good water solubility, such as liquid formulations or granules for ingestion dissolved in water.

### Prior art document

### Patent Document

Patent Document 1: JP 2017-39762 A
Patent Document 2: WO 2019/082335
Patent Document 3: JP 2012-213380 A

### Non-Patent Document

Non-Patent Document 1: Yoshida KT, Wada T, Koyama H, Mizobuchi-Fukuoka R, Naito S. Temporal and spatial patterns of accumulation of the transcript of Myo-inositol-1-phosphate synthase and phytin-containing particles during seed development in rice. Plant Physiol. 1999;119(1):65- 72.
Non-Patent Document 2: Plimmer RH, et al., Biochem J. 1913;7 (2) :157-74.

The disclosure of the prior art documents cited in this specification may be incorporated herein by reference.

### Summary of the invention

### Technical Problem

An objective of the present invention is to provide phytin with improved solubility in water, a method for producing the same, and a composition comprising the phytin.

### Solution to Problem

The present inventors diligently conducted studies to solve the above problems, and surprisingly found that phytin with molar ratios of magnesium and calcium to phytic acid within specific ranges has extremely high solubility in water, thereby completing the invention of the present disclosure. Furthermore, surprisingly, the present inventors also found that although the existing commercially available water-insoluble phytin has lower purine nucleotide metabolism inhibitory activity as compared to phytic acid, the phytin with molar ratios of magnesium and calcium to phytic acid within specific ranges has a higher purine nucleotide metabolism inhibitory activity than that of the commercially available water-insoluble phytin. And the present inventors also found that the purine nucleotide metabolism inhibitory activity of the phytin with molar ratios of magnesium and calcium to phytic acid within specific ranges is equal to or greater than that of phytic acid.

The present disclosure includes the following aspects.

[1] A phytin comprising (A) calcium and / or (B) magnesium, wherein the phytin is water-soluble.
[2] The phytin according to [1], wherein the following expressions are satisfied: 1.4x + y ≤ 5.4 (for example, 0.5 ≤ 1.4x + y ≤ 5.4, 0.8 ≤ 1.4x + y ≤ 5.4, 1 ≤ 1.4x + y ≤ 5.4), x ≥ 0, and y ≥ 0, provided that x and y are not both zero,
   wherein
   x represents the amount of substance (mol) of (A) calcium per 1.8 moles of phytic acid comprised in the phytin, and
   y represents the amount of substance (mol) of (B) magnesium per 1.8 moles of phytic acid comprised in the phytin.
[3] A phytin comprising (A) calcium and / or (B) magnesium, wherein the following expressions are satisfied: 0 ≤ x ≤ 2 (for example, 0.5 ≤ x ≤ 1.5), and 0 ≤ y ≤ 6 (for example, 0.5 ≤ y ≤ 5.5, 1 ≤ y ≤ 4.5), provided that x and y are not both zero,
   wherein
   x represents the amount of substance (mol) of (A) calcium per 1.8 moles of phytic acid comprised in the phytin, and
   y represents the amount of substance (mol) of (B) magnesium per 1.8 moles of phytic acid comprised in the phytin.
[4] The phytin according to [2] or [3], wherein the following expression is satisfied: x + y > 0.5 (for example, x + y ≥ 1.0).
[5] The phytin according to any one of [2] to [4], wherein the following expressions are satisfied: 0.3 ≤ x ≤ 1.07, and 3.9 ≤ y ≤ 4.98.
[6] The phytin according to any one of [2] to [5], wherein the following expressions are satisfied: 0.65 ≤ x ≤ 1.07, and 3.9 ≤ y ≤ 4.4.
[7] The phytin according to any one of [3] to [4], wherein the following expressions are satisfied: 0.9 ≤ x ≤ 1.1, and 3.9 ≤ y ≤ 4.1.
[8] The phytin according to any one of [1] to [7], wherein the phytin further comprises (C) potassium and / or (D) sodium.
[9] The phytin according to [8], wherein the following expressions are satisfied: 0 ≤ p ≤ 4 (for example, 0.5 ≤ p ≤ 3, 0.5 ≤ p ≤ 2, 0.5 ≤ p ≤ 1.5, 0.5 ≤ p ≤ 1.2), and 0 ≤ q ≤ 6 (for example, 0.5 ≤ q ≤ 3, 0.5 ≤ q ≤ 2, 0.5 ≤ q ≤ 1.5, 0.5 ≤ q ≤ 1.2), provided that p and q are not both zero,
   wherein
   p represents the amount of substance (mol) of (C) potassium per 1.8 moles of phytic acid comprised in the phytin, and
   q represents the amount of substance (mol) of (D) sodium per 1.8 moles of phytic acid comprised in the phytin.
[10] A composition, comprising the phytin according to any one of [1] to [9].
[11] A composition for inhibiting purine body absorption, comprising the phytin according to any one of [1] to [9].
[12] The composition according to [11], wherein the purine body absorption is purine body absorption through an intestinal tract.
[13] A composition for inhibiting purine nucleotide metabolism, comprising the phytin according to any one of [1] to [9].
[14] A composition for inhibiting phosphatase, comprising the phytin according to any one of [1] to [9].
[15]A composition for inhibiting uric acid level elevation, comprising the phytin according to any one of [1] to [9].
[16] A composition for improving blood pressure, comprising the phytin according to any one of [1] to [9].
[17] A composition for improving blood glucose level, comprising the phytin according to any one of [1] to [9].
[18] A composition for preventing hypercalciuria, comprising the phytin according to any one of [1] to [9].
[19] A composition for improving lipid level, comprising the phytin according to any one of [1] to [9].
[20] The composition according to any one of [10] to [19], wherein the composition is a solid composition (for example, powder, granule, tablet).
[21] A method for improving the solubility of phytin in water, comprising adding a calcium source (e.g., Ca(OH)₂) and a magnesium source (e.g., Mg(OH)₂) to phytic acid in the presence of water, wherein the following expressions are satisfied: 1.4x + y ≤ 5.4 (for example, 0.5 ≤ 1.4x + y ≤ 5.4, 0.8 ≤ 1.4x + y ≤ 5.4, 1 ≤ 1.4x + y ≤ 5.4), x ≥ 0, and y ≥ 0, provided that x and y are not both zero,
   wherein
   x represents the amount of substance (mol) of (A) calcium added to 1.8 moles of the phytic acid, and
   y represents the amount of substance (mol) of (B) magnesium added to 1.8 moles of the phytic acid.
[22] A method for producing phytin, comprising:
   mixing, in the presence of water, phytic acid; and a calcium source (for example, Ca(OH)₂) and / or a magnesium source (for example, Mg(OH)₂); and
   drying the resulting mixture,

   wherein the following expressions are satisfied: 1.4x + y ≤ 5.4 (for example, 0.5 ≤ 1.4x + y ≤ 5.4, 0.8 ≤ 1.4x + y ≤ 5.4, 1 ≤ 1.4x + y ≤ 5.4), x ≥ 0, and y ≥ 0, provided that x and y are not both zero,
   wherein
   x represents the amount of substance (mol) of (A) calcium per 1.8 moles of phytic acid comprised in the mixture, and
   y represents the amount of substance (mol) of (B) magnesium per 1.8 moles of phytic acid comprised in the mixture.
[23] A method for producing phytin, comprising:
   mixing, in the presence of water, phytic acid; and a magnesium source (for example, Mg(OH)₂) and / or a calcium source (for example, Ca(OH)₂); and
   drying the resulting mixture,
      wherein the following expressions are satisfied: 0 ≤ x ≤ 2 (for example, 0.5 ≤ x ≤ 1.5), and 0 ≤ y ≤ 6 (for example, 0.5 ≤ y ≤ 5.5, 1 ≤ y ≤ 4.5), provided that x and y are not both zero,
      wherein
      x represents the amount of substance (mol) of (A) calcium per 1.8 moles of phytic acid comprised in the mixture, and
      y represents the amount of substance (mol) of (B) magnesium per 1.8 moles of phytic acid comprised in the mixture.
[24] The method according to any one of [21] to [23], wherein the following expression is satisfied: x + y > 0.5 (for example, x + y ≥ 1.0).
[25] The method according to any one of [21] to [24], wherein the following expressions are satisfied: 0.3 ≤ x ≤ 1.07, and 3.9 ≤ y ≤ 4.98.
[26] The method according to any one of [21] to [25], wherein the following expressions are satisfied: 0.65 ≤ x ≤ 1.07, and 3.9 ≤ y ≤ 4.4.
[27] The method according to [23], wherein the following expressions are satisfied: 0.9 ≤ x ≤ 1.1, and 3.9 ≤ y ≤ 4.1.
[28] The method according to any one of [22] to [27], wherein in the mixing, phytic acid; and a magnesium source (for example, Mg(OH)₂) and / or a calcium source (for example, Ca(OH)₂); are mixed in the presence of water and an excipient.
[29] The method according to any one of [22] to [28], wherein in the mixing, phytic acid; a magnesium source (for example, Mg(OH)₂) and / or a calcium source (for example, Ca(OH)₂); and a potassium source (for example, KOH) and / or a sodium source (for example, NaOH); are mixed in the presence of water.
[30] The method according to any one of [22] to [29], wherein in the mixing, phytic acid; a magnesium source (for example, Mg(OH)₂) and / or a calcium source (for example, Ca(OH)₂); and a potassium source (for example, KOH) and / or a sodium source (for example, NaOH) are mixed in the presence of water and an excipient.
[31] The method according to [29] or [30], wherein the following expressions are satisfied: 0 ≤ p ≤ 4 (for example, 0.5 ≤ p ≤ 3, 0.5 ≤ p ≤ 2, 0.5 ≤ p ≤ 1.5, 0.5 ≤ p ≤ 1.2), and 0 ≤ q ≤ 6 (for example, 0.5 ≤ q ≤ 3, 0.5 ≤ q ≤ 2, 0.5 ≤ q ≤ 1.5, 0.5 ≤ q ≤ 1.2), provided that p and q are not both zero,
   wherein
   p represents the amount of substance (mol) of (C) potassium per 1.8 moles of phytic acid comprised in the mixture, and
   q represents the amount of substance (mol) of (D) sodium per 1.8 moles of phytic acid comprised in the mixture.
[32] The method according to any one of [28], [30], and [31] wherein the excipient is selected from dextrin, starches, cellulose, sugars (for example, lactose, sucrose, trehalose) and any combination thereof.
[33] A composition for inhibiting purine body absorption, comprising a water-soluble phytin comprising only calcium as a mineral component.
[34] The composition according to [33], wherein the molar ratio in the water-soluble phytin is phytic acid:calcium = 1.8:0.1 to 4.0 (mol).
[35] Use of the phytin according to any one of [1] to [9] in the manufacture of a composition for inhibiting purine body absorption, a composition for inhibiting purine nucleotide metabolism, a composition for inhibiting phosphatase, a composition for inhibiting uric acid level elevation, a composition for improving blood pressure, a composition for improving blood glucose level, a composition for improving liver function, a composition for controlling serum iron level, a composition for promoting calcium absorption, a composition for preventing hypercalciuria, or a composition for improving lipid level.
[36] A method for inhibiting purine body absorption, for inhibiting purine nucleotide metabolism, for inhibiting phosphatase, for inhibiting uric acid level elevation, for improving blood pressure, for improving blood glucose level, for improving liver function, for controlling serum iron level, for promoting calcium absorption, for preventing hypercalciuria, for improving lipid level, or for preventing deterioration of lipid level, comprising administering a composition comprising the phytin according to any one of [1] to [9].
[37] The phytin according to any one of [1] to [9] for use in inhibiting purine body absorption, inhibiting purine nucleotide metabolism, inhibiting phosphatase, inhibiting uric acid level elevation, improving blood pressure, improving blood glucose level, improving liver function, controlling serum iron level, promoting calcium absorption, preventing hypercalciuria, or improving lipid level.

Two or more of the elements described in the above [1] to [37] may be optionally selected and combined.

### Effect of Invention

The present invention provides phytin with improved solubility in water, a method for producing the same, and a composition comprising the phytin.

### Brief Description of Drawings

[FIG. 1] Figure 1 shows the results of the solubility test in Test Example 1.
   The horizontal axis indicates the molar ratio of Ca to phytic acid 1.8 mol.
   The vertical axis indicates the molar ratio of Mg to phytic acid 1.8 mol.
[FIG. 2-1] Figure 2-1 shows the results of the purine nucleotide metabolism inhibitory activity test in Test Example 2. Each column represents the mean ± standard error, and asterisks indicate P < 0.05 (vs PA, unpaired t-test).
[FIG. 2-2] Figure 2-2 shows the results of the purine nucleotide metabolism inhibitory activity test in Test Example 2. Each column represents the mean ± standard error, and asterisks indicate P < 0.05 (vs PA, unpaired t-test).

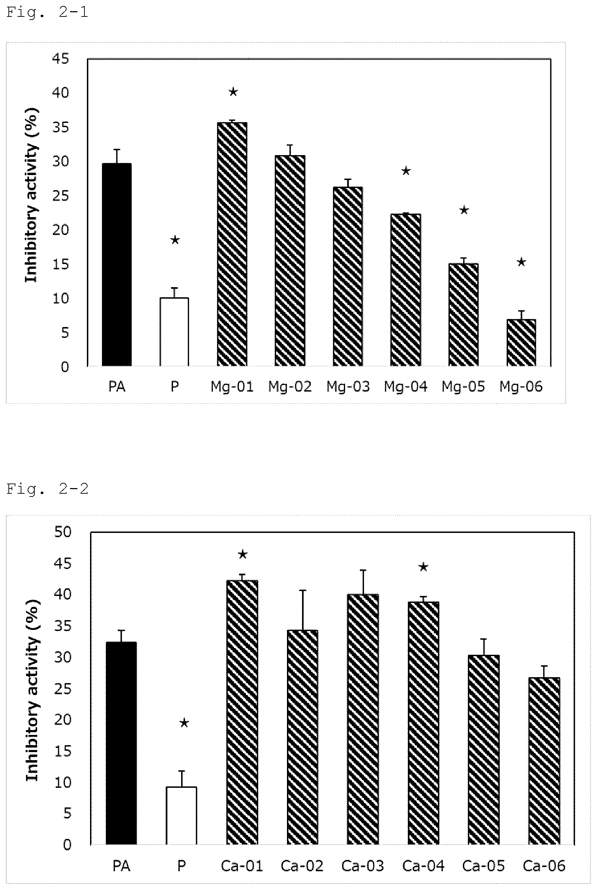

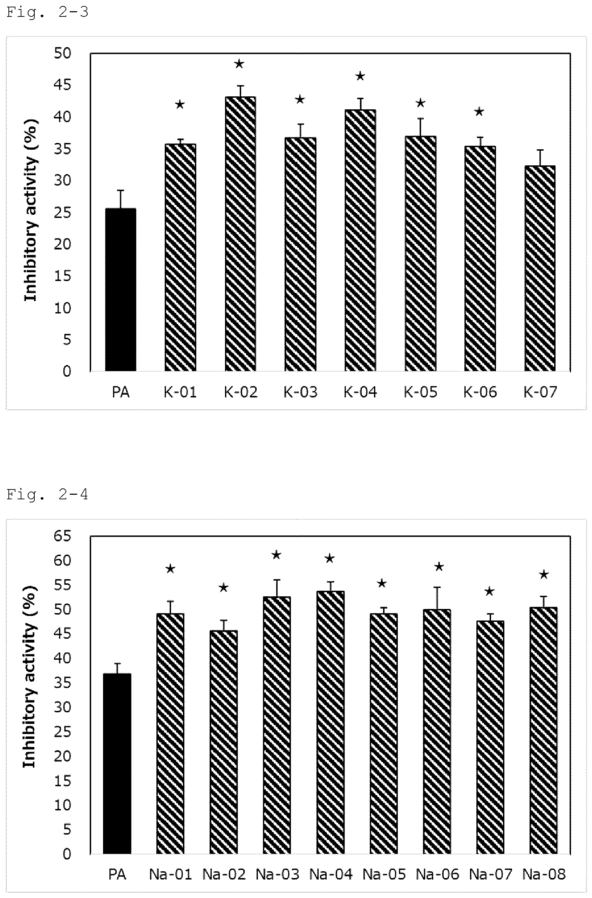

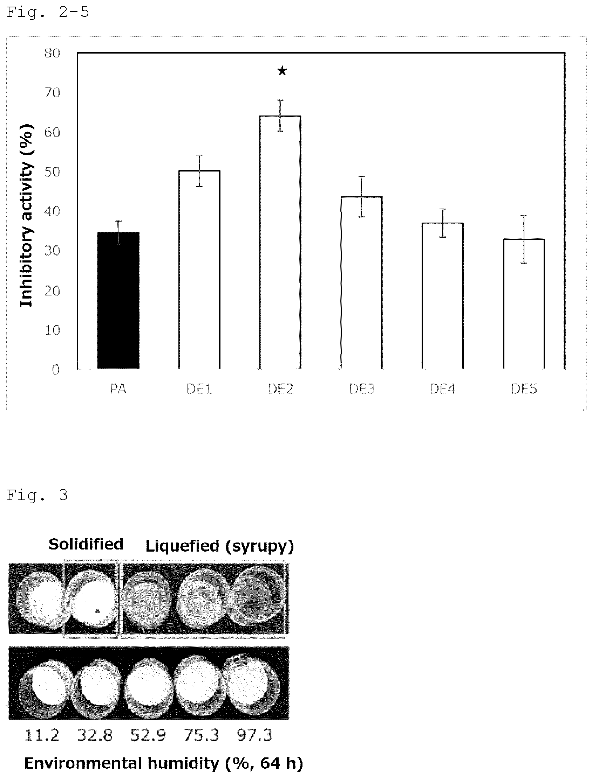

[FIG. 2-3] Figure 2-3 shows the results of the purine nucleotide metabolism inhibitory activity test in Test Example 2. Each column represents the mean ± standard error, and asterisks indicate P < 0.05 (vs PA, unpaired t-test).
[FIG. 2-4] Figure 2-4 shows the results of the purine nucleotide metabolism inhibitory activity test in Test Example 2. Each column represents the mean ± standard error, and asterisks indicate P < 0.05 (vs PA, unpaired t-test).
[FIG. 2-5] Figure 2-5 shows the results of the purine nucleotide metabolism inhibitory activity test in Test Example 2. Each column represents the mean ± standard error, and asterisks indicate P < 0.05 (vs PA, unpaired t-test).
[FIG. 3] Figure 3 shows the stability (deliquescence) test results of commercial products (phytic acid powder and phytin) in Test Example 3. Upper row: phytic acid powder. Lower row: phytin.

### Description of Embodiments

Below, embodiments of the present invention are described in detail.

In one aspect, the present invention provides a phytin with improved solubility in water, comprising (A) calcium and / or (B) magnesium (hereinafter referred to as "the phytin of the present invention").

In the present disclosure, "phytin" refers to a mineral salt of phytic acid which is a hexaphosphate ester of myo-inositol, where the mineral comprises magnesium, calcium, or both. The mineral may further comprise potassium, sodium, and the like.

In the present disclosure, "phytin with improved solubility in water" may refer to a phytin with improved solubility in water compared to a conventional phytin which comprises the mineral components at molar ratios, relative to 1.8 moles of phytic acid, of Mg:K:Na:Ca = 18.5:2.4:2:1 (mol). The conventional phytin is known to be insoluble in water. The test method of solubility in water is not particularly limited. For example, it may refer to a phytin where no turbidity or precipitation is observed by visual inspection when tested according to the method of Test Example 1 of the present application. Alternatively, it may refer to a phytin where no precipitation is observed by visual inspection when the phytin is added to water (20 to 25 °C) at 10 mg/ml, subjected to a mixing operation repeated three times in total, wherein the mixing operation comprises vortex mixing for 30 seconds followed by sonication for 60 seconds.

The phytin with improved solubility in water of the present invention may contain other mineral components (for example, potassium and sodium) in addition to calcium and magnesium as long as the phytin exhibits improved solubility in water.

In a certain embodiment, the phytin of the present invention is a water-soluble phytin.

In the present disclosure, "water-soluble phytin" refers to phytin soluble in water.

For example, "water-soluble phytin" may mean a phytin where the optical density (OD) at 600 nm measured by spectrophotometer of the mixture of the phytin is 0.008 or less, and no turbidity or precipitation is observed by visual inspection, wherein the mixture is obtained by adding the phytin to water (20 to 25 °C) at 10 mg/ml, subjected to a mixing operation repeated three times in total, wherein the mixing operation comprises vortex mixing for 30 seconds followed by sonication for 60 seconds. Alternatively, it may mean a phytin where the OD600 value is 0.008 or less, and no precipitation is observed by visual inspection, when assessed according to the method of Test Example 1 of the present application.

The water-soluble phytin of the present invention may comprise other mineral components (for example, potassium and sodium) in addition to calcium and magnesium as long as the phytin is water-soluble.

In a certain embodiment, the phytin of the present invention comprises (A) calcium and / or (B) magnesium, and the phytin satisfies the following expressions: 0 ≤ x ≤ 2, and 0 ≤ y ≤ 6, provided that x and y are not both zero,
wherein
x represents the amount of substance (mol) of (A) calcium per 1.8 moles of phytic acid comprised in the phytin, and
y represents the amount of substance (mol) of (B) magnesium per 1.8 moles of phytic acid comprised in the phytin.

In a certain embodiment, the phytin of the present invention comprises (A) calcium and / or (B) magnesium, and the phytin satisfies the following expressions: 1.4x + y ≤ 5.4, x ≥ 0, and y ≥ 0, provided that x and y are not both zero,
wherein
x represents the amount of substance (mol) of (A) calcium per 1.8 moles of phytic acid comprised in the phytin, and
y represents the amount of substance (mol) of (B) magnesium per 1.8 moles of phytic acid comprised in the phytin.

In a certain embodiment, the phytin of the present invention further comprises (C) potassium and / or (D) sodium.

In a certain embodiment, the phytin of the present invention further comprises (C) potassium and / or (D) sodium, and the phytin satisfies the following expressions: 0 ≤ p ≤ 4, and 0 ≤ q ≤ 6, provided that p and q are not both zero,
wherein
p represents the amount of substance (mol) of (C) potassium per 1.8 moles of phytic acid comprised in the phytin, and
q represents the amount of substance (mol) of (D) sodium per 1.8 moles of phytic acid comprised in the phytin.

The table below shows the amount of substance of magnesium and the amount of substance of calcium, relative to 1.8 moles of phytic acid in naturally occurring phytin contained in each plant, calculated based on the disclosure of Non-Patent Document 2 (Plimmer RH, et al., Biochem J. 1913;7 (2) :157-74).

| (mol) | Wheat | Corn | Oats | Rice | Wheat bran |
|---|---|---|---|---|---|
| Mg | 4.9 | 6.6 | 3.9 | 10.4 | 4.6 |
| Ca | 0.6 | 0.3 | 4.3 | 1.8 | 1.6 |
| Phytic acid | 1.8 | 1.8 | 1.8 | 1.8 | 1.8 |

Generally, conventionally-used phytin is a white, water-insoluble, neutral powder with good stability and non-deliquescent.

In a certain embodiment, the phytin of the present invention is not a phytin derived from wheat.

In a certain embodiment, the phytin of the present invention is not a phytin derived from natural products (for example, wheat, corn, oats, rice).

The method for producing the phytin of the present invention is not particularly limited; however, for example, the production method described below as one aspect of the present invention may be employed.

In one aspect, the present invention provides a method for producing phytin, comprising
mixing, in the presence of water, phytic acid; and a magnesium source and / or a calcium source; and
drying the resulting mixture
   (hereinafter sometimes referred to as "the production method of the present invention").

In a certain embodiment of the production method of the present invention, the following expressions are satisfied: 1.4x + y ≤ 5.4, x ≥ 0, and y ≥ 0, provided that x and y are not both zero,
wherein
x represents the amount of substance (mol) of (A) calcium per 1.8 moles of phytic acid comprised in the mixture, and
y represents the amount of substance (mol) of (B) magnesium per 1.8 moles of phytic acid comprised in the mixture.

In a certain embodiment of the production method of the present invention, the following expressions are satisfied: 0 ≤ x ≤ 2, and 0 ≤ y ≤ 6, provided that x and y are not both zero,
wherein
x represents the amount of substance (mol) of (A) calcium per 1.8 moles of phytic acid comprised in the mixture, and
y represents the amount of substance (mol) of (B) magnesium per 1.8 moles of phytic acid comprised in the mixture.

In a certain embodiment of the production method of the present invention, in the mixing, a potassium source and / or a sodium source are additionally contained and mixed.

The method for producing phytic acid to be used in the production method of the present invention is not particularly limited. For example, it may be synthesized organic-chemically or by using microorganisms, and it may also be extracts, partially purified products, or processed products of plants, microorganisms, various foods and drinks containing phytic acid. Phytic acid isolated from the extracts, partially purified products or processed products may also be used. Phytic acid may be pre-dissolved in water and then mixed with other raw materials.

In the mixing of the production method of the present invention, the concentration of phytic acid in the mixture is not particularly limited; however, for example, it may be contained at 0.01 to 2.0 pmol/mL (preferably 0.25 to 1.0 pmol/mL) as phytic acid.

In the present disclosure, a magnesium source refers to an electrolyte that can provide Mg²⁺ in water. In the production method of the present invention, the magnesium source may be used as it is or pre-dissolved in water and then mixed with other raw materials.

In a certain embodiment of the production method of the present invention, the magnesium source is Mg(OH)₂.

In the present disclosure, a calcium source refers to an electrolyte that can provide Ca²⁺ in water. In the production method of the present invention, the calcium source may be used as it is or pre-dissolved in water and then mixed with other raw materials.

In a certain embodiment of the production method of the present invention, the calcium source is Ca(OH)₂.

In the present disclosure, a potassium source refers to an electrolyte that can provide K⁺ in water. In the production method of the present invention, the potassium source may be used as it is or pre-dissolved in water and then mixed with other raw materials.

In a certain embodiment of the production method of the present invention, the potassium source is KOH.

In the present disclosure, a sodium source refers to an electrolyte that can provide Na⁺ in water. In the production method of the present invention, the sodium source may be used as it is or pre-dissolved in water and then mixed with other raw materials.

In a certain embodiment of the production method of the present invention, the sodium source is NaOH.

In a certain embodiment of the production method of the present invention, the mixing is conducted in the presence of water and an excipient. The amount of excipient is not particularly limited, but for example, the weight ratio of phytic acid:excipient = 1:0.1 to 3.0 (preferably 0.8 to 1.6, more preferably 1.1 to 1.4). The excipient to be used should preferably act to maintain the powder state of phytin.

In a certain embodiment of the production method of the present invention, "excipient" includes dextrin, starches (e.g., potato starch, corn starch), cellulose, sugars (e.g., lactose, sucrose, trehalose). One type of excipient may be used, or multiple types of excipient may be used.

In a certain embodiment of the production method of the present invention, the excipient is dextrin.

In a certain embodiment of the production method of the present invention, the pH of the mixture is 3.0 to 5.0 (preferably 3.5 to 4.5, more preferably 3.8 to 4.2), and may be adjusted if necessary with HCl or the like.

In a certain embodiment of the production method of the present invention, the mixing time in the mixing is not particularly limited, but it is preferable to mix until a clear solution is obtained, for example, for 1 to 1.5 hours.

The drying method in the drying of the production method of the present invention is not particularly limited, and may be performed by a method commonly used in the field (for example, freeze drying).

The production method of the present invention may further include additional steps such as filtration, pulverization, and the like, in addition to the mixing and the drying. In the production method of the present invention, further additives may be added to the extent that they do not adversely affect the solubility of phytin.

Hereinafter, phytin produced by the production method of the present invention may also be referred to as the phytin of the present invention.

In a certain embodiment of the present invention, the phytin of the present invention comprises (A) calcium and / or (B) magnesium in predetermined proportions. The content of (A) calcium and (B) magnesium is not particularly limited as long as the effects of the present invention, such as improved solubility, are achieved. For example, when the amount of substance of (A) calcium comprised in the phytin is x moles per 1.8 moles of phytic acid and the amount of substance of (B) magnesium comprised in the phytin is y moles per 1.8 moles of phytic acid, the following expressions are satisfied: 1.4x + y ≤ 5.4, x ≥ 0, and y ≥ 0, provided that x and y are not both zero. Furthermore preferably the following expressions are satisfied: 0.3 ≤ x ≤ 1.07, and 3.9 ≤ y ≤ 4.98.

In a certain embodiment of the present invention, the following expression is satisfied: 0.5 ≤ y ≤ 5.4.

In a certain embodiment of the present invention, the following expression is satisfied: 0.5 ≤ y ≤ 5.

In a certain embodiment of the present invention, the following expression is satisfied: 1 ≤ y ≤ 4.5.

In a certain embodiment of the present invention, the following expression is satisfied: 3.9 ≤ y ≤ 4.98.

In a certain embodiment of the present invention, the following expression is satisfied: 3.9 ≤ y ≤ 4.4.

In a certain embodiment of the present invention, the following expression is satisfied: 3.9 ≤ y ≤ 4.1.

In a certain embodiment of the present invention, the following expression is satisfied: 0.3 ≤ x ≤ 3.8.

In a certain embodiment of the present invention, the following expression is satisfied: 0.5 ≤ x ≤ 1.5.

In a certain embodiment of the present invention, the following expression is satisfied: 0.3 ≤ x ≤ 1.07.

In a certain embodiment of the present invention, the following expression is satisfied: 0.65 ≤ x ≤ 1.07.

In a certain embodiment of the present invention, the following expression is satisfied: 0.9 ≤ x ≤ 1.1.

In a certain embodiment of the present invention, the range of y excludes 4.5 ≤ ( or <) y ≤ ( or <) 5.

In a certain embodiment of the present invention, the following expressions are satisfied: 0.5 ≤ 1.4x + y ≤ 5.4, x ≥ 0, y ≥ 0, provided that x and y are not both zero.

In a certain embodiment of the present invention, the following expressions are satisfied: 0.8 ≤ 1.4x + y ≤ 5.4x ≥ 0, and y ≥ 0, provided that x and y are not both zero.

In a certain embodiment of the present invention, the following expressions are satisfied: 1 ≤ 1.4x + y ≤ 5.4, x ≥ 0, and y ≥ 0, provided that x and y are not both zero.

In a certain embodiment of the present invention, the following expressions are satisfied: 2.5 ≤ 1.4x + y ≤ 5.4, x ≥ 0, and y ≥ 0, provided that x and y are not both zero.

In a certain embodiment of the present invention, the following expression is satisfied: 0.5 ≤ p ≤ 3.

In a certain embodiment of the present invention, the following expression is satisfied: 0.5 ≤ p ≤ 2.

In a certain embodiment of the present invention, the following expression is satisfied: 0.5 ≤ p ≤ 1.5.

In a certain embodiment of the present invention, the following expression is satisfied: 0.5 ≤ p ≤ 1.2.

In a certain embodiment of the present invention, the following expression is satisfied: 0.8 ≤ p ≤ 1.2.

In a certain embodiment of the present invention, the following expression is satisfied: 0.5 ≤ q ≤ 3.

In a certain embodiment of the present invention, the following expression is satisfied: 0.5 ≤ q ≤ 2.

In a certain embodiment of the present invention, the following expression is satisfied: 0.5 ≤ q ≤ 1.5.

In a certain embodiment of the present invention, the following expression is satisfied: 0.5 ≤ q ≤ 1.2.

In a certain embodiment of the present invention, the following expression is satisfied: 0.8 ≤ q ≤ 1.2.

In a certain aspect, a composition comprising the phytin of the present invention (referred to as the composition of the present invention) is provided. By using the phytin of the present invention, compositions such as powders with improved solubility in water may be provided. Since the phytin of the present invention may have the enzyme inhibition activity equal to or greater than that of phytic acid, it may be used as a substitute for phytic acid in compositions containing phytic acid.

Phytin is traditionally recognized as a substance with abundant dietary experience and high safety, suitable for long-term and continuous intake. According to the present invention, the improvement of portability by making it into solid compositions such as powder compositions may increase convenience, which may enable longer-term and continuous intake.

In a certain embodiment, the phytin of the present invention may be non-deliquescent. Since phytic acid is deliquescent, its storage stability is low, making it difficult to use in solid formulations. On the other hand, the non-deliquescent phytin of the present invention may have excellent storage stability and activity equal to or greater than that of phytic acid, making it suitable for use as a substitute for phytic acid in formulations, especially solid formulations (such as powder, granule, tablet) that are difficult to formulate due to the deliquescence of phytic acid.

In a certain embodiment of the present invention, from the standpoint of deliquescence,
the following expression is satisfied: x + y > 0.1, preferably the following expression is satisfied: x + y > 0.3,
more preferably the following expression is satisfied: x + y > 0.5,
even more preferably the following expression is satisfied: x + y ≥ 1.0.

In a certain embodiment of the present invention, from the standpoint of purine nucleotide metabolism inhibitory activity,
the following expression is satisfied: 0 ≤ y ≤ 6,
preferably the following expression is satisfied: 0 ≤ y ≤ 5, more preferably the following expression is satisfied: 0 ≤ y ≤ 4.5.

In a certain embodiment of the present invention, from the standpoint of purine nucleotide metabolism inhibitory activity,
the following expression is satisfied: 0.5 ≤ y ≤ 6,
preferably the following expression is satisfied: 0 ≤ y ≤ 5, more preferably the following expression is satisfied: 0 ≤ y ≤ 4.5.

In a certain embodiment of the present invention, from the standpoint of purine nucleotide metabolism inhibitory activity,
the following expression is satisfied: 0 ≤ x ≤ 2,
more preferably the following expression is satisfied: 0.5 ≤ x ≤ 1.5.

In a certain embodiment of the present invention, the following expressions are satisfied: 1.4x + y ≤ 5.4, 0.9 ≤ x ≤ 1.1, 3.9 ≤ y ≤ 4.1, 0.8 ≤ p ≤ 1.2, and 0.8 ≤ q ≤ 1.2.

In a certain embodiment of the present invention, the following expressions are satisfied: x = 1, y = 4, p = 1, and q = 1.

In a certain embodiment of the present invention, from the standpoint of purine nucleotide metabolism inhibitory activity, the phytin of the present invention a water-soluble phytin that does not comprise magnesium as a mineral component.

In a certain embodiment of the present invention, the following expression is satisfied: y = 0.

In a certain embodiment of the present invention, the phytin of the present invention is a water-soluble phytin comprising only calcium as a mineral component. From the standpoint of purine nucleotide metabolism inhibitory activity, the preferred molar ratio of phytic acid:calcium = 1.8:0.1 to 4.0 (mol).

In a certain embodiment, the present invention provides a composition comprising the phytin of the present invention for inhibiting purine body absorption, for inhibiting purine nucleotide metabolism, for inhibiting phosphatase, for inhibiting uric acid level elevation, for improving blood pressure, for improving blood glucose level, for improving liver function, for improving liver function, for controlling serum iron level, or for promoting calcium absorption.

In a certain embodiment, the present invention provides a method for inhibiting purine body absorption, for inhibiting purine nucleotide metabolism, for inhibiting phosphatase, for inhibiting uric acid level elevation, for improving blood pressure, for improving blood glucose level, for improving liver function, for controlling serum iron level, or for promoting calcium absorption, comprising administering a composition (for example, a solid composition (for example, powder, granule) ) comprising the phytin of the present invention.

### <Other Components>

The phytin of the present invention may comprise a mineral component other than (A) calcium and (B) magnesium within a range that does not inhibit the effect of the present invention.

In a certain embodiment, the phytin of the present invention substantially does not comprise mineral components other than calcium, magnesium, potassium, and sodium.

### <Phytin Dosage>

The amount of phytin contained in the composition of the present invention (food and drink, pharmaceutical composition, etc.), the amount of phytin administered per administration, and the amount of phytin administered per day are not particularly limited as long as they are within the range in which the intended effect(s) are exerted, and may be selected according to the form of the composition, the number of administrations, the health condition of the subject, etc. The administration period of the composition of the present invention is not particularly limited as long as it is within the range in which the intended effect (s) are exerted, and the composition may be administered as a single dose or continuously. For example, in order to obtain a continuous effect of inhibiting purine body absorption, inhibiting purine nucleotide metabolism, inhibiting phosphatase, inhibiting uric acid level elevation, improving blood pressure, improving blood glucose level, improving liver function, controlling serum iron level, promoting calcium absorption, and the like, the composition of the present invention may be desirably ingested continuously over a long period of time, for example, 2 days, 3 days, 1 week, 10 days, 1 month, or 3 months or more.

### <Phytin Content in Compositions>

The amount of phytin comprised in the composition of the present invention may vary depending on the kind of phytin, the form of the composition, etc., and may be selected from the range of 0.1 wt% to 90 wt%, usually as the phytin, based on the total weight of the composition. Examples include 0.5 wt% to 90 wt%, 0.5 wt% to 85 wt%, 0.5 wt% to 80 wt%, 1 wt% to 70 wt%, and 1 wt% to 50 wt%. Additional examples of the upper limit of the amount of phytin comprised in the composition of the present application include 90 wt%, 85 wt%, 80 wt%, 70 wt%, 50 wt%, 30 wt%, 10 wt%, and 5 wt% as the phytin, relative to the total weight of the composition; additional examples of the lower limit of the amount of phytin comprised in the composition of the present application include 0.1 wt%, 0.5 wt%, 0.7 wt%, and 1 wt% as the phytin, relative to the total weight of the composition; and a preferred range of the amount of phytin comprised in the composition of the present invention may be indicated by a combination of the upper and lower limits. When the composition of the present invention is formulated as a solid preparation such as tablets, granules, capsules, powders, chewable tablets, etc., the amount of phytin comprised in the composition of the present invention may be selected from the range of, for example, 1 to 90 wt% based on the total weight of the composition, and preferred examples thereof include 1 wt% to 90 wt%, 5 wt% to 80 wt%, 10 wt% to 70 wt%, 50 wt% to 70 wt%.

Further examples of the upper limit of the amount of phytin comprised in the composition of the present invention when the composition of the present invention is formulated as solid preparations such as tablets, granules, capsules, powders, chewable tablets, and the like, relative to the total weight of the composition, preferably include 90 wt%, 85 wt%, 80 wt%, 70 wt%, and 50 wt%; further examples of the lower limit of the amount of phytin comprised in the composition of the present application include, relative to the total weight of the composition, preferably 1 wt%, 5 wt%, 10 wt%, and 20 wt%; and a preferred range of the amount of the phytin be indicated by a combination of the upper and lower limits.

The composition of the present invention may be such that phytin are administered, for example, 10 mg to 15 g per administration, preferably 100 mg to 8 g per administration, more preferably 300 mg to 5 g per administration as phytic acid, which may vary depending on the kind of phytin.

Examples of the lower limit of the dose of phytin per administration include 10 mg, 100 mg, 300 mg, 500 mg, and 600 mg as phytic acid; and examples of the upper limit include 15 g, 10 g, 8 g, 5 g, 3 g, 2 g, 1 g, and 600 mg as phytic acid; and a preferred range of the dose of phytin per administration may be indicated by a combination of the upper and lower limits.

The composition of the present invention may be such that phytin are administered, for example, 10 mg to 15 g per day, preferably 100 mg to 8 g per day, more preferably 300 mg to 5 g per day as phytic acid, which may vary depending on the kind of phytin. Examples of the lower limit of the dose of phytin per day include 10 mg, 100 mg, 300 mg, 500 mg, and 600 mg as phytic acid; and examples of the upper limit include 15 g, 10 g, 8 g, 5 g, 3 g, 2 g, 1 g, and 600 mg as the phytic acid; and a preferred range of the dose of phytin per day may be indicated by a combination of the upper and lower limits. The amount of phytin which may be administered per day, may be administered in a single administration or in multiple divided administrations (for example, twice, three times, four times, and five times).

### <Analytical Methods>

The amount of phytin contained in the composition of the present invention can be analyzed by generally-used analytical method(s) known to those skilled in the art. Examples thereof include, but are not limited to, an ion chromatography method and vanadomolybdate absorption spectrophotometry.

### <Intake Timing>

The timing of intake of the composition of the present invention is not particularly limited, but the composition is preferably taken during a meal or within 30 minutes before or after a meal.

### <Types of Oral Formulations>

The composition of the present invention is preferably formulated as an oral dosage form, and the formulation is not particularly limited, but may be for example, tablets, granules, capsules, powders, chewable tablets, sweets (Cookies, biscuits, chocolate confectioneries, chips, cakes, gums, candies, gummies, buns, yokan (sweet bean jelly), puddings, jellies, yogurt, ice cream, sherbet, etc.), bread, noodles, rice, cereal foods, powdered drinks, soups (powder, freeze-dry), miso soups (powder, freeze-dry), and conventional food forms.

The composition of the present invention may be formulated into orally administered formulations by adding pharmaceutically acceptable base(s), carrier(s), additive(s) usable in food, etc., in addition to phytin. It is desirable that ingredient(s) other than phytin used in the composition of the invention do not impair the stability of the phytin, and that they do not impair the intended effect(s) of the composition of the invention.

The composition of the present invention may be a food and drink or a pharmaceutical composition, and may be used as a food and drink, for example Foods with Functional Claims, Food for specified health uses, a health food, a nutritional supplement (supplement), a food for medical use, etc.

In this disclosure, a purine body is a generic term for compounds having a common structure called a purine skeleton, and plays various functions in vivo, such as transmitting genetic information as a constituent of nucleic acids. Purine body includes purine base (e.g. adenine, guanine, hypoxanthine), purine nucleoside, which is a purine base linked to a sugar, (e.g. adenosine, guanosine, and inosine), and purine nucleotide, which is a purine nucleoside linked to phosphate group(s), (e.g. adenylic acid (AMP), guanylic acid (GMP), inosinic acid (IMP)). Most of the purine bodies contained in foods are present as components of nucleic acids (nucleotide residues).

In the present invention, "purine body absorption" means that said purine bodies (purine bases, purine nucleosides, purine nucleotides, or nucleic acids containing purine nucleotide bases (e.g., oligonucleotides, polynucleotides)) are absorbed into the body, after metabolism required depending on the form of purine body.

In the present invention, "inhibiting purine body absorption" means that the amount of purine bodies (for example, dietary purine bodies) absorbed into the body (for example, absorbed into the body through the intestinal tract) is relatively reduced. The "inhibiting purine body absorption" includes, for example, inhibition of alkaline phosphatase and/or 5 'nucleotidase, which prevent conversion of purine nucleotides to purine nucleosides (This results in less purine bodies being absorbed into the body).

In the present invention, "dietary purine body (dietary purine bodies) " means purine bodies derived from an ingested food or drink.

In the present invention, "a purine body that can be absorbed through the intestinal tract" include purine base(s) and purine nucleoside(s).

In the present invention, "inhibiting purine nucleotide metabolism" means inhibiting the conversion of purine nucleotide(s) to purine nucleoside(s).

In the present invention, "inhibiting phosphatase" means inhibiting phosphatase activity involved in purine metabolism, for example includes inhibiting alkaline phosphatase and/or 5' nucleotidase.

In the present invention, the term "inhibiting uric acid level elevation" means suppressing an excessive rise in the uric acid level, and includes, for example, lowering or alleviating the rise in the serum uric acid level in a person with a high serum uric acid level (including a person whose uric acid level is in normal but a little high), and maintaining the serum uric acid level, or preventing or alleviating the rise in the serum uric acid level, in a person with a normal or low serum uric acid level.

In a certain embodiment, the present invention provides the composition for inhibiting uric acid level elevation, wherein a dose of phytin per administration is 10 mg to 15 g as phytic acid.

In the present invention, "improving blood pressure" means suppressing an excessive rise in blood pressure, and includes, for example, decreasing or alleviating blood pressure elevation in a person with a high blood pressure (including a person whose blood pressure is in normal but a little high), and maintaining blood pressure, preventing or alleviating blood pressure elevation in a person with a normal blood pressure or a little low blood pressure.

In the present invention, "improving blood glucose level" means that an excessive rise in blood glucose level is suppressed, and includes, for example, lowering or alleviating the rise in the blood glucose level in a person with a high blood glucose level (including a person whose level is in normal but a little high), and maintaining the blood glucose level and preventing or alleviating the rise in the blood glucose level in a person with a normal blood glucose level or a little low blood glucose level.

In the present invention, "improving liver function" means that liver function is improved, for example, the function may be indicated by an indicator (for example, AST: aspartate aminotransferase). For example, "improving liver function" includes lowering or alleviating the rise in blood AST level in a person with a high blood AST level (including a person whose level is in normal but a little high), and maintaining blood AST level, preventing or alleviating the rise in blood AST level in a person with a normal or a little low blood AST level.

In the present invention, "controlling serum iron level" means inhibiting an excessive increase or decrease in serum iron levels, including, for example, decreasing or alleviating the rise in serum iron level in a person with a high serum iron level (including a person whose level is in normal but a little high); increasing serum iron level or alleviating the decrease in serum iron level in a person with low serum iron level (including a person whose level is in normal but a little low); maintaining serum iron level, preventing an increase or decrease in serum iron level, or alleviating an increase or decrease in serum iron level in a person with normal serum iron level.

In the present invention, "promoting calcium absorption" means that absorption of calcium through the intestinal tract is promoted.

In the present invention, the term "promoting calcium absorption" includes an increase in the blood concentration of active vitamin D (1,25-(OH)₂-D) that promotes absorption of calcium through the intestinal tract.

A composition for inhibiting purine body absorption, a composition for inhibiting purine nucleotide metabolism, a composition for inhibiting phosphatase, a composition for inhibiting uric acid level elevation, a composition for improving blood pressure, a composition for improving blood glucose level, a composition for improving liver function, a composition for controlling serum iron level, or a composition for promoting calcium absorption, of the present invention may be formulated as a formulation to be used for these intended uses (inhibiting purine nucleotide metabolism, inhibiting phosphatase, inhibiting uric acid level elevation, improving blood pressure, improving blood glucose level, improving liver function, controlling serum iron level, or promoting calcium absorption). The intended use of the formulation may be indicated on the product itself, packaging, instructions, pamphlets, containers, advertising, advertising materials for the sales floor such as POP, other documents, electromagnetic methods (Internet, etc.), etc. For example, the following products stating the intended use or expressions that suggest / are reminiscent of the intended use: drugs (including quasi-drugs); Food for specified health uses, food with nutrient function claims, food with functional claims, etc., for which indications have been approved by designated organizations; ordinary foods and drinks are also within the scope of the present invention.

For example, with respect to a composition for inhibiting purine body absorption or a composition for inhibiting uric acid level elevation of the present invention, foods and drinks, etc., in which expressions such as "For those who are concerned about purine bodies in meals", "fight purine bodies", "For those who tend to eat meals with a lot of purine bodies", "For those concerned about postprandial uric acid levels", etc., which suggest / are reminiscent of inhibiting purine body absorption or inhibiting uric acid level elevation, are also within the scope of the present invention.

With regard to a composition for improving blood pressure of the present invention, foods and drinks, etc. in which expressions such as "suitable for a person with a little high blood pressure", "support healthy blood pressure", "capable of lowering a little high blood pressure", "For a person who is concerned about blood pressure", "maintain normal blood pressure", "improve blood pressure", and the like which suggest / are reminiscent of improving blood pressure are described are also included in the scope of the present invention.

With regard to a composition for improving blood glucose level of the present invention, foods and drinks, etc., in which expressions such as "Suitable for a person who is concerned about their blood glucose level", "moderate the rise in blood glucose level", "keep a little high blood glucose level in normal", "for a person with a little high blood glucose level", etc., which suggest / are reminiscent of improving blood glucose level are described are also included in the scope of the present invention.

With regard to a composition for improving liver function of the present invention, foods and drinks, etc. in which expressions such as "Maintaining a healthy liver" and "for people with a higher figure of liver function indicator" which suggest / are reminiscent of improving liver function are described are also included in the scope of the present invention.

With regard to a composition for controlling serum iron level of the present invention, foods and drinks, etc. in which expressions such as "For a person who is concerned about iron deficiency" and "For the prevention of anemia" which suggest / are reminiscent of controlling serum iron level are described are also included in the scope of the present invention.

With regard to a composition for promoting calcium absorption of the present invention, foods and drinks, etc. in which expressions such as "contribute to bone health", "maintenance of bone components", "help bone metabolism", "maintain strong bones", "To prevent osteoporosis", and the like which suggest / are reminiscent of promoting calcium absorption are described are also included in the scope of the present invention.

### <Subject>

The subject of administration of the composition of the present invention is not particularly limited, but is preferably human. The composition of the present invention may be taken by a healthy person for daily health in addition to a person being concerned about uric acid level, blood pressure, blood glucose level, liver function, anemia or bone health.

### Example

### Manufacturing example 1

A commercially available phytic acid solution (product name: phytic acid (IP6), TSUNO Food Industrial co., ltd.) 108 mL (containing 75 g of phytic acid) was mixed with 1362 mL of Milli-Q water, and stirred well for a few minutes at a water temperature of 40-60 °C. A Magnetic stirrer was used for stirring (the same applies below). To the resulting diluted phytic acid solution was added 8.4 g of Ca(OH)₂, and the mixture was stirred well until completely Ca(OH)₂ is dissolved at a water temperature of 35 to 55 °C for 10-40 minutes. Subsequently, 26.5g of Mg(OH)₂ was added thereto, and the mixture was stirred well for 10 to 40 minutes at a water temperature of 30 to 50 °C. Finally, 30 mL of 3.79 M KOH-NaOH aqueous solution was added in small portions thereto, and the mixture was stirred well for 10 to 40 minutes at a water temperature of 25 to 45 °C. To the resulting mixture was added a diluted phytic acid solution which was prepared by using the commercially available phytic acid solution (product name: phytic acid (IP6), TSUNO Food Industrial co., ltd.) 86 mL (containing 60 g of phytic acid) and 1114 mL of Milli-Q water. Then the mixture was adjusted to pH 4.0 ± 0.2 with hydrochloric acid, and then stirred well for 30 to 120 minutes. To the resulting mixture was added 84.0 g dextrin, and the mixture was stirred well for 30 to 90 minutes. The mixture was then placed in a suitable container, and freeze-dried, and the resulting phytin product contains phytin wherein the molar ratios, relative to 1.8 moles of phytic acid, of Mg:K:Na:Ca = 4:1:1:1 (mol).

### Test Example 1 Solubility

To determine the solubility of different types of phytin, phytic acid and minerals were mixed at predetermined molar ratios in water, and the turbidity of the resulting mixture was examined. The turbidity corresponds to the solubility of the corresponding phytin.

### <Test Method>

### (Sample Preparation)

At room temperature of 20 to 25 °C, Ca(OH)₂ (Inoue Calcium Corporation) as a calcium source, Mg(OH)₂ (Tomita Pharmaceutical Co.,Ltd.) as a magnesium source, KOH (NIPPON SODA CO., LTD.) as a potassium source, NaOH (Tosoh Corporation) as a sodium source were placed in a 50 mL PP tube (IWAKI) at the molar ratios of minerals shown in the tables below. Then a commercially available phytic acid solution (product name: phytic acid (IP6), TSUNO Food Industrial co., ltd.) 0.426 mL (containing 0.296 g of phytic acid) was placed therein. Distilled water (Otsuka Pharmaceutical Factory, Inc.) was added thereto to make up the volume to 30 mL. After stirring for 30 seconds using a vortex mixer, ultrasonication (ASONE) was performed for 60 seconds. This operation of stirring and ultrasonication was repeated three times in total.

### (Turbidity Measurement and Visual Inspection)

Each sample obtained above was stirred for 30 seconds, and turbidity measurement (Molecular Devices) was conducted according to Clarity of Solution Test in Japan's Specifications and Standards for Food Additives (9th Edition, 2018). The absorbance at 600 nm for the reference solution "Almost clear" was 0.008.

The sample was visually observed to determine the presence or absence of phytin precipitation.

The symbol "o" indicates "No precipitation".

The symbol "×" indicates "Precipitation present".

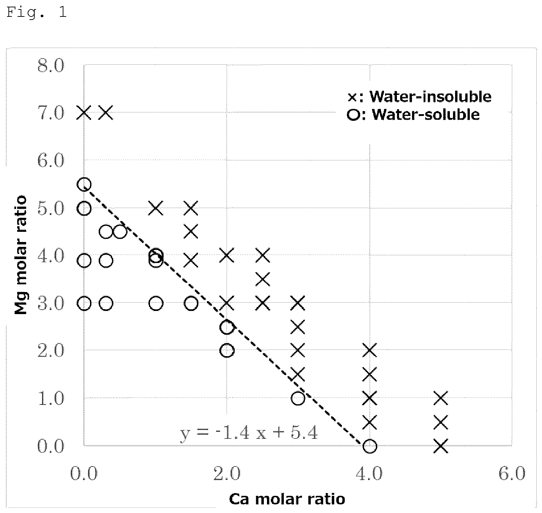

If the absorbance was 0.008 or less and no precipitation was observed visually, it was classified as "water-soluble." Otherwise, it was classified as "water-insoluble." The results are shown in the tables below and Figure 1.

| Table 1-1 | | | | | | | |
|---|---|---|---|---|---|---|---|
| Sample No. | Molar ratio to 1.8 mol of phytic acid | | | | OD600 | Visually | Solubility |
| | Mg | Ca | Na | K | | | |
| R1 | 3.0 | 0.0 | 1 | 1 | 0.003 | ○ | Water-soluble |
| R2 | 3.9 | 0.0 | 1 | 1 | 0.006 | o | Water-soluble |
| R3 | 5.0 | 0.0 | 1 | 1 | 0.002 | ○ | Water-soluble |
| AG12 | 5.0 | 0.0 | 1 | 1 | 0.002 | ○ | Water-soluble |
| AG11 | 5.5 | 0.0 | 1 | 1 | 0.003 | o | Water-soluble |
| R5 | 7.0 | 0.0 | 1 | 1 | 0.233 | × | Water-insoluble |
| R6 | 3.0 | 0.3 | 1 | 1 | 0.001 | o | Water-soluble |
| R7 | 3.9 | 0.3 | 1 | 1 | 0.001 | ○ | Water-soluble |
| AG19 | 4.5 | 0.3 | 1 | 1 | 0.000 | o | Water-soluble |
| R10 | 7.0 | 0.3 | 1 | 1 | 0.897 | × | Water-insoluble |
| AG18 | 4.5 | 0.5 | 1 | 1 | 0.001 | o | Water-soluble |
| R11 | 3.0 | 1.0 | 1 | 1 | 0.001 | o | Water-soluble |
| R12 | 3.9 | 1.0 | 1 | 1 | 0.006 | o | Water-soluble |
| R13 | 4.0 | 1.0 | 1 | 1 | 0.002 | ○ | Water-soluble |
| R15 | 5.0 | 1.0 | 1 | 1 | 0.064 | × | Water-insoluble |
| R16 | 3.0 | 1.5 | 1 | 1 | 0.002 | ○ | Water-soluble |
| R17 | 3.9 | 1.5 | 1 | 1 | 0.012 | × | Water-insoluble |
| R19 | 4.5 | 1.5 | 1 | 1 | 0.948 | × | Water-insoluble |
| R20 | 5.0 | 1.5 | 1 | 1 | 1.933 | × | Water-insoluble |

| Table 1-2 | | | | | | | |
|---|---|---|---|---|---|---|---|
| Sample No. | Molar ratio to 1.8 mol of phytic acid | | | | OD600 | Visually | Solubility |
| | Mg | Ca | Na | K | | | |
| AG7 | 2.0 | 2.0 | 1 | 1 | 0.002 | ○ | Water-soluble |
| AG21 | 2.5 | 2.0 | 1 | 1 | 0.003 | ○ | Water-soluble |
| R25 | 4.0 | 2.0 | 1 | 1 | 0.005 | × | Water-insoluble |
| AG3 | 3.0 | 2.5 | 1 | 1 | 0.707 | × | Water-insoluble |
| R29 | 3.5 | 2.5 | 1 | 1 | 0.002 | × | Water-insoluble |
| R30 | 4.0 | 2.5 | 1 | 1 | 0.022 | × | Water-insoluble |
| R31 | 1.0 | 3.0 | 1 | 1 | 0.001 | o | Water-soluble |
| R32 | 1.5 | 3.0 | 1 | 1 | 0.001 | × | Water-insoluble |
| R33 | 2.0 | 3.0 | 1 | 1 | 0.001 | × | Water-insoluble |
| R34 | 2.5 | 3.0 | 1 | 1 | 0.002 | × | Water-insoluble |
| AG4 | 3.0 | 3.0 | 1 | 1 | 2.691 | × | Water-insoluble |
| R36 | 0.0 | 4.0 | 1 | 1 | 0.006 | ○ | Water-soluble |
| R37 | 0.5 | 4.0 | 1 | 1 | 0.001 | × | Water-insoluble |
| AG6 | 1.0 | 4.0 | 1 | 1 | 3.303 | × | Water-insoluble |
| R39 | 1.5 | 4.0 | 1 | 1 | 0.002 | × | Water-insoluble |
| R40 | 2.0 | 4.0 | 1 | 1 | 0.005 | × | Water-insoluble |
| AG5 | 0.0 | 5.0 | 1 | 1 | 0.173 | × | Water-insoluble |
| R42 | 0.5 | 5.0 | 1 | 1 | 0.002 | × | Water-insoluble |
| R43 | 1.0 | 5.0 | 1 | 1 | 0.002 | × | Water-insoluble |

### Discussion

All phytin samples satisfying the following expression: 1.4x + y ≤ 5.4 were water-soluble. In the above expression, x represents the amount of substance (mol) of calcium per 1.8 moles of phytic acid, and y represents the amount of substance (mol) of magnesium per 1.8 moles of phytic acid.

### Manufacturing Example 2

Each sample obtained in Test Example 1 was freeze-dried to obtain each phytin (powder) with the molar ratios shown in Table 1.

### Test Example 2: Effect of Mineral Molar Ratios in Phytin on Purine Nucleotide Metabolism Inhibitory Activity

### (Sample Preparation)

Ca(OH)₂ (Inoue Calcium Corporation) as a calcium source, Mg(OH)₂ (Tomita Pharmaceutical Co.,Ltd.) as a magnesium source, KOH (NIPPON SODA CO., LTD.) as a potassium source, NaOH (Tosoh Corporation) as a sodium source were placed in a 50 mL PP tube (IWAKI) at molar ratios of the minerals shown in Table 2. Then a commercially available phytic acid solution (product name: phytic acid (IP6), TSUNO Food Industrial co., ltd.) 0.426 mL (containing 0.296 g of phytic acid) was placed therein. Distilled water (Otsuka Pharmaceutical Factory, Inc.) was added thereto to make up the volume to 30 mL. The mixture was stirred for 30 seconds using a vortex mixer, followed by ultrasonication (ASONE) for 60 seconds to prepare a test solution containing phytin with various mineral compositions.

As a positive control, a test solution containing a commercially available phytin (phytin (IP6), TSUNO Food Industrial co., ltd.) was prepared at the same concentration of phytic acid as the other test solutions.

### (Turbidity Measurement and Visual Inspection)

Each test solution obtained above was stirred for 30 seconds, and turbidity measurement (Molecular Devices) was conducted according to Clarity of Solution Test in Japan's Specifications and Standards for Food Additives (9th Edition, 2018). The absorbance at 600 nm for the reference solution "Almost clear" was 0.008.

The test solution was visually observed to determine the presence or absence of phytin precipitation.

If the absorbance was 0.008 or less and no precipitation was observed visually, it was classified as "water-soluble." Otherwise, it was classified as "water-insoluble."

The results are shown in Table 2.

### (Test method of Purine Nucleotide Metabolism Inhibitory Activity)

A 100 mg/mL of solution of rat small intestine powder (Clare Japan, Inc.) in the assay buffer was used as an enzyme solution. Inosine monophosphate (Sigma-Aldrich) was diluted with the assay buffer to the concentration of 9 mM to prepare a substrate solution. Enzymatic reaction was performed using a 96 well plate (Thermo Fisher Scientific) in the following manner. The test solution (100 pL/well), the substrate solution (100 µL/well) and the enzyme solution (100 µL/well) were mixed, and the enzyme reaction was performed at 37 °C for 30 minutes. A 20 µL aliquot of the reaction mixture was transferred to multi-screen HTS HV (Merck Millipore), and the reaction was stopped by adding the stop solution (0.33 M HClO₄, 180 pL/well). Centrifugation (1080 x g for 10 minutes at room temperature) was performed, and the filtrate was subjected to high performance liquid chromatography (HPLC).

The analytical conditions of HPLC are shown below.
Detector: SPD-M30A (Shimadzu Corporation)
Column: COSMOSIL PAQ (4.6 mm I.D. × 150 mm, NACALAI TESQUE, INC.)
Mobile phase: 50 mM KH₂PO₄ (pH7.5)
Flow rate: 1.0 mL/min. Detection wavelength: 254 nm, Column
temperature: 35 °C
Sample injection volume: 10 µL, pH: 6.4

The results are shown in FIGs. 2-1 to 2-5.

| Table 2 | | | | | |
|---|---|---|---|---|---|
| Sample No. | Molar ratio to 1.8 mol of phytic acid | | | | Water solubility |
| | Mg | Ca | Na | K | |
| Phytic acid (PA) | 0 | 0 | 0 | 0 | Water-soluble |
| Phytin (P) | 18.5 | 2.4 | 2 | 1 | Water-insoluble |
| (Commercial product) | | | | | |
| Mg-01 | 4 | 1 | 1 | 1 | Water-soluble |
| Mg-02 | 6 | 1 | 1 | 1 | Water-insoluble |
| Mg-03 | 8 | 1 | 1 | 1 | Water-insoluble |
| Mg-04 | 12 | 1 | 1 | 1 | Water-insoluble |
| Mg-05 | 16 | 1 | 1 | 1 | Water-insoluble |
| Mg-06 | 20 | 1 | 1 | 1 | Water-insoluble |
| Ca-01 | 4 | 1 | 1 | 1 | Water-soluble |
| Ca-02 | 4 | 2 | 1 | 1 | Water-insoluble |
| Ca-03 | 4 | 4 | 1 | 1 | Water-insoluble |
| Ca-04 | 4 | 8 | 1 | 1 | Water-insoluble |
| Ca-05 | 4 | 12 | 1 | 1 | Water-insoluble |
| Ca-06 | 4 | 20 | 1 | 1 | Water-insoluble |
| Na-01 | 4 | 1 | 0 | 1 | Water-soluble |
| Na-02 | 4 | 1 | 1 | 1 | Water-soluble |
| Na-03 | 4 | 1 | 2 | 1 | Water-soluble |
| Na-04 | 4 | 1 | 4 | 1 | Water-insoluble |
| Na-05 | 4 | 1 | 6 | 1 | Water-insoluble |
| Na-06 | 4 | 1 | 12 | 1 | Water-insoluble |
| Na-07 | 4 | 1 | 16 | 1 | Water-insoluble |
| Na-08 | 4 | 1 | 20 | 1 | Water-insoluble |
| K-01 | 4 | 1 | 1 | 0 | Water-soluble |
| K-02 | 4 | 1 | 1 | 1 | Water-soluble |
| K-03 | 4 | 1 | 1 | 4 | Water-insoluble |
| K-04 | 4 | 1 | 1 | 8 | Water-insoluble |
| K-05 | 4 | 1 | 1 | 12 | Water-insoluble |
| K-06 | 4 | 1 | 1 | 16 | Water-insoluble |
| K-07 | 4 | 1 | 1 | 20 | Water-insoluble |
| DE1 | 4 | 1 | 1 | 1 | Water-soluble |
| DE2 | 0 | 1 | 0 | 0 | Water-soluble |
| DE3 | 4 | 0 | 0 | 0 | Water-soluble |
| DE4 | 0 | 0 | 1 | 0 | Water-soluble |
| DE5 | 0 | 0 | 0 | 1 | Water-soluble |

### Discussion:

The comparative tests of enzyme inhibitory activity compared to phytic acid (product name: phytic acid (IP6), TSUNO Food Industrial co., ltd.) were conducted under a condition where the enzyme inhibitory activity of phytic acid was around 30% and the inhibitory activity of phytin was significantly lower compared to phytic acid.

Mg-01, which contains 4 moles of magnesium per 1.8 moles of phytic acid showed a significant increase in the activity. On the other hand, Mg-02 to Mg-06 comprising 6, 8, 12, 16, and 20 moles of magnesium respectively, showed a significant decrease in inhibitory activity in inverse proportion to the molar ratio of magnesium.

Ca-01 to 04 comprising 1, 2, 4, and 8 moles of calcium, respectively, per 1.8 moles of phytic acid showed the increased activity.

The results of Na-01 to Na-08 comprising 0, 1, 2, 4, 6, 12, 16, and 20 moles of sodium respectively, per 1.8 moles of phytic acid suggest that the addition of sodium has no effect on the inhibitory activity.

The results of K-01 to K-07 comprising 0, 1, 4, 8, 12, 16, and 20 moles of potassium respectively, per 1.8 moles of phytic acid suggest that the addition of potassium has no effect on the inhibitory activity.

DE2 comprising only 1 mole of calcium as a mineral component per 1.8 moles of phytic acid showed a significantly increased inhibitory activity of about 1.9 times higher as compared to phytic acid.

DE3 comprising only 4 moles of magnesium as a mineral component per 1.8 moles of phytic acid showed an increased inhibitory activity of about 1.3 times higher as compared to phytic acid.

DE4 comprising only 1 mole of sodium as a mineral component per 1.8 moles of phytic acid showed no change in the inhibitory activity as compared to phytic acid.

DE5 comprising only 1 mole of potassium as a mineral component per 1.8 mol of phytic acid showed no change in the inhibitory activity as compared to phytic acid.

Water-soluble phytins, Mg-01, Ca-01, K-02, Na-02, DE1 (these have the same composition), K-01, Na-01, Na-03, DE2, and DE3 showed the enzyme inhibitory activity higher compared to phytic acid.

### Test Example 3: Stability of Commercial Products (Deliquescence)

A commercially available phytic acid powder product (product name: Powdered phytic acid, FUSO CHEMICAL CO., LTD.) and a commercially available phytin (product name: Phytin (IP6), TSUNO Food Industrial co., ltd., powder) were left for 64 hours at each humidity level of 11.2 %, 32.8 %, 52.9 %, 75.3 %, and 97.3 %, and their states were observed visually.

The test humidity environments were created by placing each saturated solution of lithium chloride (equilibrium relative humidity (ERH) : 11.2), magnesium chloride (ERH: 32.8), magnesium nitrate (ERH: 52.9), sodium chloride (ERH: 75.3), and potassium sulfate (ERH: 97.3) in a desiccator, installing a thermo-hygrometer, and allowing it to stand at 25 °C.

As a result, the phytic acid powder solidified at a humidity of 32.8 %, and liquefied like starch syrup at a humidity of 52.9 % or higher. On the other hand, no change was observed in phytin under all humidity conditions.

### Test Example 4 Stability (Deliquescence)

### (Sample Preparation)

Ca(OH)₂ (Inoue Calcium Corporation) as a calcium source, Mg(OH)₂ (Tomita Pharmaceutical Co.,Ltd.) as a magnesium source, KOH (NIPPON SODA CO., LTD.) as a potassium source, NaOH (Tosoh Corporation) as a sodium source were placed in a 50 mL PP tube (IWAKI) at the molar ratios of minerals shown in Table 3. Then a commercially available phytic acid solution (product name: phytic acid (IP6), TSUNO Food Industrial co., ltd.) 0.426 mL (containing 0.296 g of phytic acid) was placed therein. Distilled water (Otsuka Pharmaceutical Factory, Inc.) was added thereto to make up the volume to 30 mL. The mixture was stirred for 30 seconds using a vortex mixer, followed by ultrasonication (ASONE) for 60 seconds. This operation of stirring and ultrasonication was repeated three times in total. Then the mixture was adjusted to pH 4.0 ± 0.2 with hydrochloric acid and pre-frozen at -80 °C. The pre-frozen mixture was freeze-dried by using a freeze dryer to provide a completely powdered phytin. The completely powdered phytin was used as a sample.

As positive controls, a sample of phytic acid (powder) prepared following the above procedure without adding any mineral component, and a commercially available phytic acid powder product (product name: Powdered phytic acid, FUSO CHEMICAL CO., LTD.) were used. As a negative control, a commercially available phytin (phytin (IP6), TSUNO Food Industrial co., ltd.) was used.

### (Test Method)

A saturated aqueous solution of sodium chloride was placed in a desiccator and allowed to stand at a temperature of 25 °C, so that the humidity became 75.3%. 200 mg of each sample was placed in a petri dish (MS-11350: SUMILON), placed in a desiccator, and left to stand for 64 hours. According to the results of Test Example 1, Sample Nos. 2, 3, 6-9 and 12-21 are water-soluble.
After the exposure, visual observation was conducted. In the column of deliquescence in Table 3,
"Deliquesced" refers to a change where the sample completely liquefied to become starch syrup-like;
"Partially-deliquesced" refers to a change where a part of the sample deliquesced or solidified.
"No change" refers to a no change in state of the sample.

### (Results)

The results are shown in Table 3.

The phytic acid sample and phytic acid powder, positive controls, deliquesced, becoming syrupy.

In contrast, the phytin samples 1 to 21 showed either no change or only partially deliquesced or partially solidified, indicating that they are more stable regarding deliquescence compared to phytic acid.

| Table 3 | | | | | |
|---|---|---|---|---|---|
| Sample | Molar ratio to 1.8 mol of phytic acid | | | | Deliquescence |
| | Mg | Ca | Na | K | |
| Phytic acid | 0 | 0 | 0 | 0 | Deliquesced |
| 1 | 6 | 0 | 1 | 1 | No change |
| 2 | 4 | 0 | 1 | 1 | No change |
| 3 | 4 | 1 | 1 | 1 | No change |
| 4 | 4 | 2 | 1 | 1 | No change |
| 5 | 4 | 4 | 1 | 1 | No change |
| 6 | 2 | 0 | 1 | 1 | No change |
| 7 | 2 | 2 | 1 | 1 | No change |
| 8 | 1 | 1 | 1 | 1 | No change |
| 9 | 0 | 2 | 1 | 1 | No change |
| 10 | 0 | 4 | 1 | 1 | No change |
| 11 | 0 | 6 | 1 | 1 | No change |
| 12 | 4 | 1 | 1 | 1 | No change |
| 13 | 2 | 0 | 1 | 1 | No change |
| 14 | 1 | 1 | 1 | 1 | No change |
| 15 | 2 | 0 | 1 | 1 | No change |
| 16 | 1 | 0 | 1 | 1 | No change |
| 17 | 0.5 | 0.5 | 1 | 1 | No change |
| 18 | 0 | 1 | 1 | 1 | No change |
| 19 | 0.5 | 0 | 1 | 1 | Partially-deliquesced |
| 20 | 0.25 | 0.25 | 1 | 1 | Partially-deliquesced |
| 21 | 0 | 0.5 | 1 | 1 | Partially-deliquesced |
| Phytin (Commercial product) | 18.5 | 2.4 | 2 | 1 | No change |
| Phytic acid powder (Commercial product) | 0 | 0 | 0 | 0 | Deliquesced |

### Test Example 5: Long-Term Storage Stability

### (Sample Preparation)

The long-term storage stability test of a water-soluble phytin product prepared according to Manufacturing example 1 (molar ratio to 1.8 mol of phytic acid: Mg:Ca:Na:K=4:1:1:1 (mol), dextrin concentration: 67%) was conducted. Specifically, the water activity of the phytin product was measured over time for up to three months under a storage condition of 25 °C and 60% humidity using a stability test apparatus (ETAC LABONIC LX330, Kusumoto Chemicals, Ltd.), or under a storage condition of 40 °C and 75% humidity using a constant temperature chamber (Incubator IS82, Yamato Scientific co., ltd.). The water activity was determined at a measuring temperature of 25 °C using a measuring instrument (LabMster-aw Basic, Novasina). The sample's appearance was visually observed.

The storage conditions, the results of water activity and appearance are shown in Table 4. The water activity of the water-soluble phytin product before the storage under high humidity conditions was 0.020. When stored at 25 °C and 60% humidity, it increased slightly over time to 0.027-0.030, and when stored at 40 °C and 75% humidity, it increased slightly over time to 0.034-0.047. In general, microorganisms do not proliferate in foods with the water activity of 0.5 or less. That is, the water activity of 0.5 or less means a state of no moisture adsorption. The results of water activity, which are less than one-tenth of the moisture activity of 0.5 or less indicating non-hygroscopic conditions, suggest that the moisture absorption of the phytin product is little. Additionally, regarding the appearance, it remained as a white powder without deliquescing. From these results, it was determined that the phytin product may exist stably in powder form over a long period.

| Table 4 | | | |
|---|---|---|---|
| Storage condition | Storage period (month) | Water activity | Appearance |
| 4 °C | 0 | 0.020 | White powder |
| 25 °C, 60 %RH | 0.5 | 0.027 | |
| | 1 | 0.025 | |
| | 3 | 0.030 | No change in appearance |
| 40 °C, 75 %RH | 0.5 | 0.034 | |
| | 1 | 0.036 | |
| | 3 | 0.047 | |

### Industrial Applicability

The phytin of the present invention is particularly useful in formulations requiring good water solubility, such as liquid formulations and granules for ingestion dissolved in water at the time of use.

## Claims

1. A phytin comprising (A) calcium and / or (B) magnesium, wherein the phytin is water-soluble.

2. The phytin according to Claim 1, wherein the following expressions are satisfied: 1.4x + y ≤ 5.4, x ≥ 0, and y ≥ 0, provided that x and y are not both zero,
wherein
x represents the amount of substance (mol) of (A) calcium per 1.8 moles of phytic acid comprised in the phytin, and
y represents the amount of substance (mol) of (B) magnesium per 1.8 moles of phytic acid comprised in the phytin.

3. A phytin comprising (A) calcium and / or (B) magnesium, wherein the following expressions are satisfied: 0 ≤ x ≤ 2, and 0 ≤ y ≤ 6, provided that x and y are not both zero,
wherein
x represents the amount of substance (mol) of (A) calcium per 1.8 moles of phytic acid comprised in the phytin, and
y represents the amount of substance (mol) of (B) magnesium per 1.8 moles of phytic acid comprised in the phytin.

4. The phytin according to Claim 2 or 3, wherein the following expression is satisfied: x + y > 0.5.

5. The phytin according to any one of Claims 2 to 4, wherein the following expressions are satisfied: 0.3 ≤ x ≤ 1.07, and 3.9 ≤ y ≤ 4.98.

6. The phytin according to any one of Claims 2 to 5, wherein the following expressions are satisfied: 0.65 ≤ x ≤ 1.07, and 3.9 ≤ y ≤ 4.4.

7. The phytin according to any one of Claims 3 to 4, wherein the following expressions are satisfied: 0.9 ≤ x ≤ 1.1, and 3.9 ≤ y ≤ 4.1.

8. The phytin according to any one of Claims 1 to 7, wherein the phytin further comprises (C) potassium and / or (D) sodium.

9. The phytin according to Claim 8, wherein the following expressions are satisfied: 0 ≤ p ≤ 4, and 0 ≤ q ≤ 6, provided that p and q are not both zero,
wherein
p represents the amount of substance (mol) of (C) potassium per 1.8 moles of phytic acid comprised in the phytin, and
q represents the amount of substance (mol) of (D) sodium per 1.8 moles of phytic acid comprised in the phytin.

10. A composition, comprising the phytin according to any one of Claims 1 to 9.

11. A composition for inhibiting purine body absorption, comprising the phytin according to any one of Claims 1 to 9.

12. The composition according to Claim 11, wherein the purine body absorption is purine body absorption through an intestinal tract.

13. A composition for inhibiting purine nucleotide metabolism, comprising the phytin according to any one of Claims 1 to 9.

14. A composition for inhibiting phosphatase, comprising the phytin according to any one of Claims 1 to 9.

15. A composition for inhibiting uric acid level elevation, comprising the phytin according to any one of Claims 1 to 9.

16. A composition for improving blood pressure, comprising the phytin according to any one of Claims 1 to 9.

17. A composition for improving blood glucose level, comprising the phytin according to any one of Claims 1 to 9.

18. A composition for preventing hypercalciuria, comprising the phytin according to any one of Claims 1 to 9.

19. A composition for improving lipid level, comprising the phytin according to any one of Claims 1 to 9.

20. The composition according to any one of Claims 10 to 19, wherein the composition is a solid composition.

21. A method for improving the solubility of phytin in water, comprising adding a calcium source and a magnesium source to phytic acid in the presence of water, wherein the following expressions are satisfied: 1.4x + y ≤ 5.4, x ≥ 0, and y ≥ 0, provided that x and y are not both zero,
wherein
x represents the amount of substance (mol) of (A) calcium added to 1.8 moles of the phytic acid, and
y represents the amount of substance (mol) of (B) magnesium added to 1.8 moles of the phytic acid.

22. A method for producing phytin, comprising:
mixing, in the presence of water, phytic acid; and a calcium source and / or a magnesium source; and
drying the resulting mixture,
wherein the following expressions are satisfied: 1.4x + y ≤ 5.4, x ≥ 0, and y ≥ 0, provided that x and y are not both zero,
wherein
x represents the amount of substance (mol) of (A) calcium per 1.8 moles of phytic acid comprised in the mixture, and
y represents the amount of substance (mol) of (B) magnesium per 1.8 moles of phytic acid comprised in the mixture.

23. A method for producing phytin, comprising:
mixing, in the presence of water, phytic acid; and a magnesium source and / or a calcium source; and
drying the resulting mixture,
wherein the following expressions are satisfied: 0 ≤ x ≤ 2, and 0 ≤ y ≤ 6, provided that x and y are not both zero,
wherein
x represents the amount of substance (mol) of (A) calcium per 1.8 moles of phytic acid comprised in the mixture, and
y represents the amount of substance (mol) of (B) magnesium per 1.8 moles of phytic acid comprised in the mixture.

24. The method according to any one of Claims 21 to 23, wherein the following expression is satisfied: x + y > 0.5.

25. The method according to any one of Claims 21 to 24, wherein the following expressions are satisfied: 0.3 ≤ x ≤ 1.07, and 3.9 ≤ y ≤ 4.98.

26. The method according to any one of Claims 21 to 25, wherein the following expressions are satisfied: 0.65 ≤ x ≤ 1.07, and 3.9 ≤ y ≤ 4.4.

27. The method according to Claim 23, wherein the following expressions are satisfied: 0.9 ≤ x ≤ 1.1, and 3.9 ≤ y ≤ 4.1.

28. The method according to any one of Claims 22 to 27, wherein in the mixing, phytic acid; and a magnesium source and / or a calcium source; are mixed in the presence of water and an excipient.

29. The method according to any one of Claims 22 to 28, wherein in the mixing, phytic acid; a magnesium source and / or a calcium source; and a potassium source and / or a sodium source; are mixed in the presence of water.

30. The method according to any one of Claims 22 to 29, wherein in the mixing, phytic acid; a magnesium source and / or a calcium source; and a potassium source and / or a sodium source are mixed in the presence of water and an excipient.

31. The method according to Claim 29 or 30, wherein the following expressions are satisfied: 0 ≤ p ≤ 4, and 0 ≤ q ≤ 6, provided that p and q are not both zero,
wherein
p represents the amount of substance (mol) of (C) potassium per 1.8 moles of phytic acid comprised in the mixture, and
q represents the amount of substance (mol) of (D) sodium per 1.8 moles of phytic acid comprised in the mixture.

32. The method according to any one of Claims 28, 30, and 31 wherein the excipient is selected from dextrin, starches, cellulose, sugars and any combination thereof.

33. A composition for inhibiting purine body absorption, comprising a water-soluble phytin comprising only calcium as a mineral component.

34. The composition according to Claim 33, wherein the molar ratio in the water-soluble phytin is phytic acid:calcium = 1.8:0.1 to 4.0 (mol).

35. Use of the phytin according to any one of Claims 1 to 9 in the manufacture of a composition for inhibiting purine body absorption, a composition for inhibiting purine nucleotide metabolism, a composition for inhibiting phosphatase, a composition for inhibiting uric acid level elevation, a composition for improving blood pressure, a composition for improving blood glucose level, a composition for improving liver function, a composition for controlling serum iron level, a composition for promoting calcium absorption, a composition for preventing hypercalciuria, or a composition for improving lipid level.

36. A method for inhibiting purine body absorption, for inhibiting purine nucleotide metabolism, for inhibiting phosphatase, for inhibiting uric acid level elevation, for improving blood pressure, for improving blood glucose level, for improving liver function, for controlling serum iron level, for promoting calcium absorption, for preventing hypercalciuria, for improving lipid level, or for preventing deterioration of lipid level, comprising administering a composition comprising the phytin according to any one of Claims 1 to 9.

37. The phytin according to any one of Claims 1 to 9 for use in inhibiting purine body absorption, inhibiting purine nucleotide metabolism, inhibiting phosphatase, inhibiting uric acid level elevation, improving blood pressure, improving blood glucose level, improving liver function, controlling serum iron level, promoting calcium absorption, preventing hypercalciuria, or improving lipid level.
